(19) **Europäisches Patentamt / European Patent Office / Office européen des brevets**

(11) **EP 4 354 449 A1**

(12) **EUROPEAN PATENT APPLICATION**

(43) Date of publication:
**17.04.2024 Bulletin 2024/16**

(21) Application number: **23202542.9**

(22) Date of filing: **09.10.2023**

(51) International Patent Classification (IPC):
**G16H 20/10** (2018.01)    **G16H 20/17** (2018.01)
**A61B 5/00** (2006.01)

(52) Cooperative Patent Classification (CPC):
**G16H 20/10; A61B 5/14532; A61B 5/4839;
A61B 5/7275; G16H 20/17**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **11.10.2022 FR 2210407**

(71) Applicant: **Diabeloop
38000 Grenoble (FR)**

(72) Inventors:
• **MADROLLE, Stéphanie, Marine
38000 GRENOBLE (FR)**
• **GALLEGOS, Alexandre
75002 PARIS (FR)**
• **TOURKI, Yousra
92800 PUTEAUX (FR)**
• **ROMERO-UGALDE, Hector
38420 LE VERSOUD (FR)**

(74) Representative: **Pellegri, Michel Pascal Romain
Cabinet Vulpelex
52 Rue Montgolfier
69006 Lyon (FR)**

(54) **CONTROL DEVICE FOR DETERMINING A RECOMMENDATION VALUE OF A CONTROL PARAMETER OF A FLUID INFUSION DEVICE**

(57)     A control device (30) for determining a recommendation value of a control parameter of a fluid infusion device (20). The control device (30) comprises a retrieving unit (32) configured to retrieve user data, a segmentation unit (34), the segmentation unit (34) configured to create a plurality of time segments in order to group user data, a segment correction unit (36) configured to create a segment correction value for each time segment and a period correction unit (38) configured to create an updated correction coefficient for a future determined period of time. The control device (30) also comprises a recommendation unit (40) configured to determine the recommendation value.

[Fig. 1]

**Description**

## FIELD OF THE INVENTION

The present invention relates to a device and method for determining a glycemia cyclic temporal behaviour over a given cyclic period of time, and a medical system including such device.

## BACKGROUND OF THE INVENTION

In the field of healthcare, and more precisely in the field of treating diabetes, it is known to evaluate the concentration of blood glucose, also called glycemia, to inject a quantity of insulin as a function of the measured concentration.

Recently, so-called "closed-loop" systems were developed, where a processor is programmed to evaluate a volume rate of insulin to be injected, based on patient-related data and/or time-based data, such as past and/or present measures of glycemia, and to control the injection of insulin based on this evaluation. In addition, the processor can be programmed to evaluate a volume of insulin to be injected in some special circumstances, in particular meals and/or physical activity. The quantity can be injected to the patient, subject to the patient's approval. Such systems are also called "semi closed-loop" systems because of the necessary declaration by the patient of some of these special circumstances.

The time-based data is often used to predict the future concentration of glycemia. This prediction is then used to calculate the quantity of insulin having to be injected in order to maintain the concentration of blood glucose in acceptable range.

An incorrect prediction of the future blood glucose can lead to an irrelevant calculated quantity of insulin to be injected, leading to a concentration of blood glucose in unacceptable intervals, where the patient may be in hypoglycemia and/or hyperglycemia.

One main drawback of this method is that the method does not enable taking into account a general behaviour of the glycemia, influenced, for example, by the patient's habits. This leads to a prediction of a future glycemia lacking accuracy.

Parameter changes in hybrid closed loops are mainly offloaded to the diabetologist or the patient. Some parameters are complex and consequences of changes are hard to infer. Analysis of the previous settings can be rigid and not take into account physical activity or unannounced meals for example. Some transitory parameter changes can be applied, but not in an automatic way.

Patients may also have continuous gradual variations or transitory variations of their physiology, due to external factors like illness, infections or acute stress. Physiological models can't take into account those changes without changing the parameters of the models.

The invention thus aims to answer at least partially of the above presented technical problems.

## BRIEF SUMMARY OF THE INVENTION

Thus, the invention relates to a control device for determining a recommendation value of a control parameter of a fluid infusion device, wherein the control device comprises:

- a retrieving unit, the retrieving unit being configured to retrieve user data, each data of the user data having a timestamp and the user data being related to a unique user during a determined period of time, the user data comprising at least:

    a plurality of amounts of a drug infused to the unique user;
    a plurality of physiological values of the unique user;
    a plurality of estimated values;
    at least one correction coefficient;

- a segmentation unit, the segmentation unit being configured to create a plurality of time segments in order to group user data at least based on their timestamp;
- a segment correction unit, the segment correction unit being configured to create a segment correction value for each time segment, wherein the segment correction unit is being configured to create the segment correction values based on at least one estimated value of the plurality of estimated values and at least one physiological value of the plurality of physiological values during a time segment of the plurality of time segments;
- a period correction unit, the period correction unit being configured to create an updated correction coefficient for a future determined period of time, wherein the period correction unit is configured to create the updated correction coefficient based at least on a segment correction value of the segment correction values and the at least one correction coefficient of the determined period of time;

- a recommendation unit, the recommendation unit being configured to determine the recommendation value during the future determined period of time, wherein the recommendation unit is being configured to determine the recommendation value at least based on the updated correction coefficient, one of the plurality of amounts of a drug infused to the unique user, one of the plurality of physiological values of the unique user and one of the plurality of estimated values.

According to an embodiment, an estimated value is a value estimated at least based on a physiological value of the plurality of physiological values of the unique user, or an amount of a drug infused to the unique user of the plurality of amounts of a drug infused to the unique user. According to the present invention, retrieving could also mean calculating, and/or receiving a calculated value from another unit.

According to an embodiment, the fluid infusion device could be of any kind such as a pump or a pen for example.

According to an embodiment, the fluid infusion device is configured to infuse drugs to a user, wherein the drugs could be of any kind such as insulin or glucagon and preferably insulin.

According to an embodiment, the drug of the plurality of amounts of a drug could be of any kind such as insulin or glucagon and preferably insulin.

According to an embodiment, the control device for determining a recommendation value of a control parameter of a fluid infusion device is a control device for determining a recommendation value of a control parameter of a fluid infusion device for a diabetic user.

According to an embodiment, the most recent data of the user data is not older than a fraction of the length of time of the determined period of time and preferably a fifth of the length of time of the determined period of time. Such a configuration allows to have recent data that correspond to the state of the unique user as much as possible.

According to an embodiment, the physiological values of the plurality of physiological values are glycemia values.

According to the present invention, glycemia must be understood as blood glucose value, estimated blood glucose value, interstitial glucose value or estimated interstitial glucose value.

According to an embodiment, some correction coefficients of at least one correction coefficient have the same value.

According to an embodiment, some estimated values of the plurality of estimated values have the same value.

According to an embodiment, there is one estimated value of the plurality of estimated values per determined period of time.

According to the present invention, the term "being configured to retrieve" means "retrieving".

According to the present invention, the term "being configured to create" means "creating".

According to the present invention, the determined period of time represents a period of time of the past while the future determined period of time represents a period of time of the future in view of the determined period of time and therefore, a part of the future determined period of time could be in the present.

According to an embodiment, the recommendation unit is configured to determine a recommendation value of a control parameter of the fluid infusion device based on the user data and preferably the plurality of amounts of a drug infused to the unique user, the plurality of physiological values of the unique user and a plurality of estimated values.

An updated correction coefficient allows the recommendation unit to accurately determine the recommendation value as the updated correction coefficient helps the recommendation unit to adapt to the unique user changes over time.

An updated correction coefficient based at least on the at least one correction coefficient of the determined period of time allows the recommendation unit to accurately determine the recommendation value without implying too steep changes on the recommendation value. Too steep changes must be avoided as it could potentially reduce the time in which the unique user is in range, or in other words, the time in which the unique user physiological value such as glycemia is in an acceptable range.

An updated correction coefficient based at least on a segment correction value of the segment correction values allows the recommendation unit to accurately determine the recommendation value as each segment correction values can be created differently, time segment by time segment or depending on a specific interest of a time segment for example.

According to an embodiment the retrieving unit is configured to add physiological values having a neutral value to the plurality of physiological values if the number of physiological values of the plurality of physiological values is under a predetermined number in order to obtain at least a number of physiological values equal to the predetermined number.

According to the present invention, a predetermined number means a predetermined threshold.

Such a configuration allows the segment correction unit to create a segment correction value for each time segment despite the lack of physiological values of the plurality of physiological values.

According to an embodiment, the segment correction unit is being configured to create a segment correction value for each time segment wherein the segment correction value would have been able to allow the recommendation unit to determine the recommendation value such as said recommendation value is sensibly equal to a value of drug that should have been injected to the specific user during a considered time segment in order to obtain at least one physiological value of the plurality of physiological values sensibly on at least one estimated of the plurality of estimated values. The at least one physiological value and the at least one estimated value corresponding to the considered time segment, and wherein the segment correction value is used by the recommendation unit as the updated correction coefficient. Such a characteristic allows to obtain a segment correction value that would have been sensibly a perfect fit to the unique user in order to obtain physiological values on the estimated values and therefore allows the recommendation unit to determine more accurately the recommendation value. According to a specific embodiment, the segment correction value correspond to an average of a plurality of subsegment correction values, each subsegment correction value corresponding to a sensibly perfect fit between a physiological value of the plurality of physiological values and an estimated value of the plurality of estimated values.

According to an embodiment, the segment correction unit is configured to use the plurality of amounts of drug infused to the unique user in order to estimate the Insulin On Board (IOB) wherein said drug infused to the unique user is insulin.

According to an embodiment wherein the plurality of physiological values are blood glucose values, the segment correction unit is configured to use the plurality of physiological values of the unique user and at least one meal size in order to estimate a Carbohydrates On Board (COB). The COB represents the carbohydrates ingested but not yet digested, which means not visible in the blood and not raising the glycemia yet.

The segment correction unit is also configured to determine a difference between the blood glycemia at the start of a time segment and the blood glycemia at the end of the same time segment and a difference between the COB at the start of the time segment and the COB at the end of the same time segment and add the ingested carbohydrates in order to determine the consumed carbohydrates. Such a configuration allows to determine the amount of insulin consumed in order to compensate for said differences.

According to an embodiment, the determined period of time is split into three types of time segments:

- meal period, a meal period is a period which start at the beginning of a carbohydrates intakes and end under conditions such as the deviation or a median deviation or an average deviation between an estimated value of the plurality of estimated values and a physiological value of the plurality of physiological values is only due to insulin and not carbohydrates intakes;
- resting period, a resting period is a period during which the IOB is low and the blood glucose is only influenced by basal insulin; and
- filling period, a filling period is a period that is not a meal period nor a resting period, nor a physical period corresponding to a period of physical activity.

According to an embodiment, some types of time segments or even time segments can be ignored and therefore not considered. Such a configuration allows to avoid meaningless data, potentially night time, regular dataless periods or biassed physiological behaviour caused by specific medical treatment such as dialysis for example.

According to an embodiment, the segment correction unit is also configured to estimate an amount of insulin consumed during a time segment as follow:

$$Insulin\ consumed = (Bo + Ba - BR) + (IOBs - IOBe)$$

wherein:

- Bo is the amount of bolus insulin infused in the unique user during the time segment and is comprised in the plurality of amounts of a drug infused to the unique user;
- Ba is the amount of basal insulin infused in the unique user during the time segment and is comprised in the plurality of amounts of a drug infused to the unique user;
- BR is a reference value, or reference basal, corresponding to a basal insulin amount used to compensate for the natural blood glucose production of the unique user;
- IOBs is the IOB at a start of the considered time segment;
- IOBe is the IOB at an end of the considered time segment;

Having an insulin consumed estimated based on *Bo + Ba - BR* allows to estimate said insulin consumed taking into account the insulin infused in order to compensate for ingested carbohydrates but not the insulin consumed in order to compensate for the natural blood glucose of the unique user and therefore allows the segment correction unit to more accurately create a segment correction value for of a time segment of an interest segment list related to meal periods.

Having an insulin consumed estimated based on (*IOBs - IOBe*) allows to estimate said insulin consumed based on the insulin ingested in order to compensate for ingested carbohydrates taking into account all previous insulin injection of the unique user and the long term temporal effect of such injections and therefore allows the segment correction unit to more accurately create a segment correction value for a time segment of an interest segment list related to meal periods.

According to an embodiment, a segment correction value and an updated correction coefficient from a previous implementation of the method are considered to be estimated values of the plurality of estimated values. Therefore, an updated correction coefficient can correspond to an ISF for example.

According to an embodiment the user data also comprises a plurality of meals and wherein the recommendation unit is being configured to determine the recommendation value also based on at least one of the plurality of meals.

Such a configuration allows the recommendation unit to determine an accurate recommendation value as the meal has an important impact on the physiological values.

According to the present invention, a meal comprises a meal size corresponding to an amount of ingested carbohydrates in a meal.

According to an embodiment, the user data also comprises a plurality of meal types, one meal type per meal or per meal size. Such a configuration allows the recommendation unit to determine an accurate recommendation value as the meal type has an important impact on the physiological values. According to the present invention, a meal type could be of any kind such as a rescue carb, a fat meal, a lunch, a dinner, a breakfast or a combination thereof for example.

According to an embodiment the segmentation unit is also being configured to create an interest segment list, the interest segment list comprising time segments from the plurality of time segments and being selected based on a specific interest and wherein the segment correction unit is configured to create a segment correction value only for each time segment of the interest segment list.

Such a characteristic allows to create an interest segment list comprising time segments that are the most useful to accurately create an updated correction coefficient and therefore to accurately determine a recommendation value of the control parameter.

According to an embodiment, a specific interest could be of any sort such as the time of the day, meal period, post meal period, inactivity, or physical activity time for example.

To accurately determine a recommendation value based on the updated correction coefficient, one of the plurality of amounts of a drug infused to the unique user, one of the plurality of physiological values of the unique user and one of the plurality of estimated values, it is important to create the updated correction coefficient based on time segments of the interest segment list. Therefore, according to an embodiment wherein the correction coefficient and the updated correction coefficient are coefficients used to compensate for carbohydrates intakes of the unique user, the time segments of the interest segment list correspond to meal periods. In this embodiment, each segment correction value is equal to the consumed insulin divided by the consumed carbohydrates. According to an embodiment, the updated correction coefficient cannot be lower than 0,02 nor higher than 0,3. Such a characteristic allows to reduce the risk of having a recommendation value too low or too high.

According to an embodiment, the period correction unit is being configured to create an updated correction coefficient based at least on the at least one correction coefficient of the determined period of time and a median of all the segment correction values.

Such a characteristic allows to minimise the importance of outliers segment correction values and therefore increase the accuracy of the created updated correction coefficient and therefore accurately determine a recommendation value of the control parameter.

According to an embodiment, the period correction unit is being configured to create an updated correction coefficient based at least on the at least one correction coefficient of the determined period of time and an average of all the segment correction values.

Such a characteristic allows to create an updated correction coefficient that allows to accurately determine a recommendation value of the control parameter that is typical of the period.

According to an embodiment, the time segments of the plurality of time segments have different lengths of time.

Such a characteristic allows to create time segments with different lengths depending on the unique user's action during said time segments. For example, the inactivity time segments will have a length of time equal to the inactivity periods while the post meal segments could have a predefined length of time. Time segments with different lengths of time allows to accurately determine segment correction values and therefore accurately determine the updated correction coefficient and the determined period of time.

According to an embodiment, the segment correction unit is being configured to create a segment correction value for each time segment based on a deviation between at least one estimated value of the plurality of estimated values and at least one physiological value of the plurality of physiological values during a time segment of the plurality of time segments, then normalise the deviation by an aggressiveness factor.

According to the present invention, the aggressiveness factor is included in the user data and corresponds to a user specific factor applied to the recommendation value. For example, the aggressiveness factor is different depending on the meal type in order to adapt to the unique user reaction depending on said meal type.

Such a characteristic allows to circumvent aggressiveness factor and therefore allows to accurately determine segment correction values and therefore accurately determine the updated correction coefficient and recommendation value.

According to an embodiment, the period correction unit is being configured to create an updated correction coefficient for a future determined period of time based at least on a segment correction value of the segment correction values and the at least one correction coefficient of the determined period of time, and wherein the updated correction coefficient is equal to the at least one correction coefficient, plus or minus a first predetermined percentage.

According to an embodiment, the period correction unit is being configured to create an updated correction coefficient for a future determined period of time based at least on a segment correction value of the segment correction values and the at least one correction coefficient of the determined period of time, and wherein the updated correction coefficient is equal to the at least one correction coefficient, plus or minus 10% to 20% and preferably 10%.

Such a characteristic allows to create an updated correction coefficient that is not too different from the correction coefficient and therefore does not change too much the determination of the recommendation value while still having a strong effect on said determination of the recommendation value. Such a characteristic also allows to obtain an updated correction coefficient that is not too much impacted by the user data having a timestamp inside the determined period of time and thus gradually converge towards its optimal value.

According to the present invention, "the updated correction coefficient is equal to the at least one correction coefficient to plus or minus a first predetermined percentage" means that the period correction unit is being configured to create an updated correction coefficient for a future determined period of time based at least on a segment correction value of the segment correction values and the at least one correction coefficient of the determined period of time, and if the updated correction coefficient appears to be, for example, equal to 160% of the at least one correction coefficient, the period correction unit is being configured to amend the updated correction coefficient in order for said updated correction coefficient to be equal to the at least one correction coefficient, plus or minus a first predetermined percentage.

According to an embodiment, the period correction unit is being configured to create an updated correction coefficient for a future determined period of time according to the following formula:

$$Ucc = (A * Cc) + ((1 - A) * Mcv)$$

wherein:

- Ucc is the updated correction coefficient;
- A is a weighting coefficient;
- Cc is the at least one correction coefficient; and
- Mcv is the at least one median segment correction value corresponding to the median of the segment correction values.

Such a characteristic allows to create an updated correction coefficient based on a plurality of past correction coefficients as the correction coefficient was an updated correction coefficient during the past determined period of time. Having an updated correction based on a plurality of past correction coefficients allows to to smooth the updated correction coefficient changes and therefore to avoid too steep changes.

According to an embodiment, the period correction unit is being configured to create an updated correction coefficient for a future determined period of time using any type of smoothing formula taking into account both correction coefficient and updated correction coefficient such as the smoothing formula described above.

The present invention also relates to a method for determining the recommendation value of a control parameter of the fluid infusion device, wherein the method is implemented by a control device as described above, wherein the method comprises the steps of:

- retrieving the user data;
- creating a plurality of time segments in order to group user data at least based on their timestamp;
- creating a segment correction value for each time segment, the segment correction values is based at least one estimated value of the plurality of estimated values and at least one physiological value of the plurality of physiological values during a time segment of the plurality of time segments;
- creating the updated correction coefficient for a future determined period of time, wherein the updated correction coefficient is based at least on a segment correction value of the segment correction values and the at least one correction coefficient of the determined period of time; and

- determining the recommendation value during the future determined period of time, the recommendation value is determined at least based on the updated correction coefficient, one of the plurality of amounts of a drug infused to the unique user, one of the plurality of physiological values of the unique user and one of the plurality of estimated values.

The definitions, embodiments and technical effects previously described obviously apply, mutatis mutandis, to the method for determining the recommendation value.

According to an embodiment, the step of creating a segment correction value is performed only if the number of physiological values of the plurality of physiological values is equal or greater to a predetermined number.

Such a configuration allows to stop the method before the step of determining the recommendation value and therefore increase the security of said method as no recommendation value is generated based on only few physiological values.

According to an embodiment, the steps are performed at a regular interval.

Such a configuration allows to determine the recommendation value more accurately interval after interval while adapting to the specific user changes.

According to an embodiment, the time length of said interval is sensibly equal to the length of the determined period of time. According to the present invention, sensibly means equal, more or less 5%. Such a configuration allows to keep the coherence between each iteration without having to use a weight for each iteration.

According to the present invention, when the steps are performed again, the updated correction coefficient becomes the correction coefficient while the future determined period of time becomes the determined period of time.

According to an embodiment wherein the steps are performed at a regular interval, the step of creating the updated correction coefficient implemented by the period correction unit consists of creating an updated correction coefficient for a future determined period of time based at least on a segment correction value of the segment correction values and the at least one correction coefficient of the determined period of time, and wherein the updated correction coefficient is equal to the first correction coefficient of the at least one correction coefficient, plus or minus 50%. Such a characteristic allows to create an updated correction coefficient that is not too different from the first correction coefficient of the at least one correction coefficient and therefore does not change too much the determination of the recommendation value while still having a strong effect on said determination of the recommendation value.

The invention also relates to a computer program product comprising instructions which, when the program is executed by a computer, cause the computer to carry out the steps of the method described above.

The various non-incompatible aspects defined above can be combined.

## BRIEF DESCRIPTION OF THE DRAWINGS

Embodiments of the invention will be described below with reference to the drawings, described briefly below:

[Fig. 1] shows a schematic view of a control device according to one embodiment of the invention; and
[Fig. 2] shows steps of a method for determining the recommendation value of a control parameter of the fluid infusion device according to one embodiment of the invention.

## DETAILED DESCRIPTION OF THE INVENTION

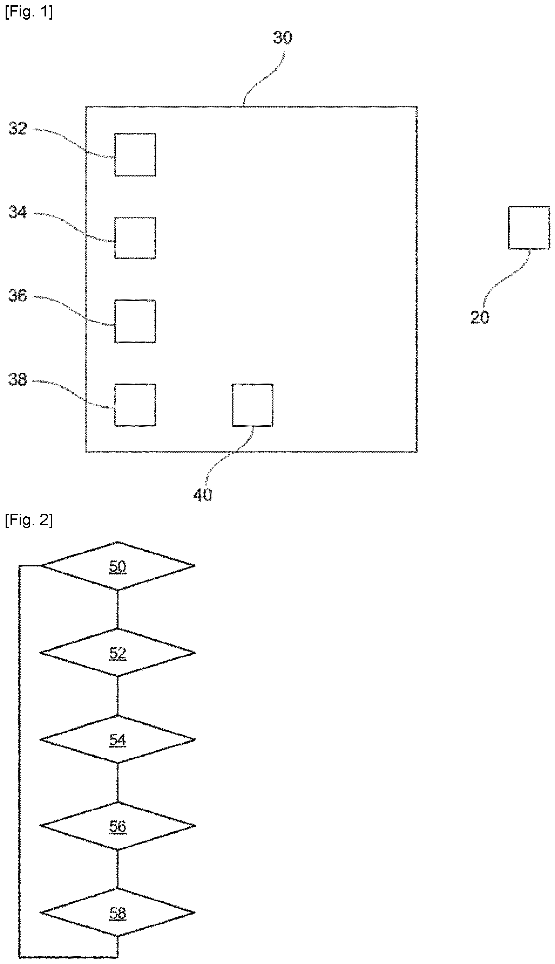

Figure 1 shows a control device 30 for determining a recommendation value of a control parameter of a fluid infusion device 20 configured to infuse insulin or glucagon such as an insulin pump or an insulin pen for a diabetic user. The control device 30 comprises a retrieving unit 32 retrieving user data. Each data of the user data has a timestamp, and the user data are related to a unique user during a determined period of time. The user data comprises at least:

a plurality of amounts of a drug infused to the unique user;
a plurality of physiological values of the unique user such as blood glucose values;
a plurality of meal sizes, as meal sizes represent the amount of carbohydrates comprised in a meal;
A plurality of meal types, as meal types could correspond to rescue carb, a fat meal, a lunch, a dinner, a breakfast or a combination thereof for example.
a plurality of estimated values; and
at least one correction coefficient, and one correction coefficient per determined period of time.

The retrieving unit 32 also adds physiological values having a neutral value to the plurality of physiological values if the number of physiological values of the plurality of physiological values is under a predetermined number in order to obtain at least a number of physiological values equal to the predetermined number. Such a configuration allows a segment correction unit 36 to create a segment correction value for each time segment despite the lack of physiological values of the plurality of physiological values.

According to the present invention, retrieving could also mean calculating, and/or receiving a calculated value from another unit.

An estimated value is a value estimated at least based on a physiological value of the plurality of physiological values of the unique user, or an amount of a drug infused to the unique user of the plurality of amounts of a drug infused to the unique user. Further embodiments are described below.

The control device 30 comprises a segmentation unit 34, the segmentation unit 34 creating a plurality of time segments in order to group user data at least based on their timestamp. The determined period of time is split by the segmentation unit 34 into three types of time segments:

meal period, a meal period is a period which start at the beginning of a carbohydrates intakes and end under conditions such as a deviation or a median deviation or an average deviation between an estimated value of the plurality of estimated values and a physiological value of the plurality of physiological values is only due to insulin and not carbohydrates intakes;

resting period, a resting period is a period during which the IOB is low and the blood glucose is only influenced by basal insulin; and

filling period, a filling period is a period that is not a meal period nor a resting period, nor a physical period corresponding to a period of physical activity.

Such a configuration allows working on specific time segments and therefore increases the accuracy of the recommendation value.

Some types of time segments or even time segments can be ignored and therefore not considered. Such a configuration allows to avoid meaningless data, potentially night time, regular dataless periods or biassed physiological behaviour caused by specific medical treatment such as dialysis for example.

The segmentation unit 34 also creates an interest segment list, the interest segment list comprising time segments from the plurality of time segments and being selected based on a specific interest, preferably segment type, and wherein the segment correction unit 36 is configured to create a segment correction value only for each time segment of the interest segment list. Such a characteristic allows to create an interest segment list comprising time segments that are the most useful to accurately create an updated correction coefficient and therefore to accurately determine a recommendation value of the control parameter.

The time segments of the plurality of time segments have different lengths of time, such a characteristic allows to create time segments with different lengths depending on the unique user's action during said time segments. For example, the inactivity time segments will have a length of time equal to the inactivity periods while the post meal segments could have a predefined length of time. Time segments with different lengths of time allows to accurately determine a recommendation value.

The control device 30 comprises the segment correction unit 36, the segment correction unit 36 creating a segment correction value for each time segment based on at least one estimated value of the plurality of estimated values and at least one physiological value of the plurality of physiological values during a time segment of the plurality of time segments.

The segment correction unit 36 is also configured to estimate an amount of insulin consumed during a time segment as follow:

$$Insulin\ consumed = (Bo + Ba - BR) + (IOBs - IOBe)$$

wherein:

- Bo is the amount of bolus insulin infused in the unique user during the time segment and is comprised in the plurality of amounts of a drug infused to the unique user;
- Ba is the amount of basal insulin infused in the unique user during the time segment and is comprised in the plurality of amounts of a drug infused to the unique user;
- BR is a reference value, or reference basal, corresponding to a basal insulin amount used to compensate for the natural blood glucose production of the unique user;
- IOBs is the Insulin On Board at a start of the considered time segment; and
- IOBe is the IOB at an end of the considered time segment;

Having an insulin consumed estimated based on *Bo* + *Ba* - *BR* allows to estimate said insulin consumed taking into account the insulin consumed in order to compensate for ingested carbohydrates but not the insulin consumed in order to compensate for the natural blood glucose of the unique user and therefore allows the segment correction unit 36 to more accurately create a segment correction value for of a time segment of an interest segment list related to meal periods.

Having an insulin consumed estimated based on (*IOBs - IOBe*) allows to estimate said insulin consumed based on the insulin ingested in order to compensate for ingested carbohydrates and therefore allows the segment correction unit 36 to more accurately create a segment correction value for a time segment of an interest segment list related to meal periods.

According to an embodiment, the segment correction unit 36 is also configured to estimate an insulin normalised during a time segment as follow:

$$Insulin\ normalised\ =\ Insulin\ consumed\ +\frac{(BGe\ -\ BGs)}{ISF}$$

Wherein:

- *BGs is the blood glucose at the start of the considered time segment; and*
- *BGe is the blood glucose at the end of the considered time segment.*

Having an insulin normalised estimated based on $\frac{(BGe\ -\ BGs)}{ISF}$ allows to estimate said insulin estimated as if BGe - BGs is a negative value, that means that a certain quantity of insulin, in addition to the injected insulin, has been consumed and must be taken into account. If BGe

BGs is a positive value, that means that some of the injected insulin has not been consumed and must be taken into account.

The segment correction unit 36 then creates a segment correction value based on the estimated insulin consumed or the estimated insulin normalised.

According to an embodiment, the segment correction unit 36 is being configured to create a segment correction value for each time segment deviation between at least one estimated value of the plurality of estimated values and at least one physiological value of the plurality of physiological values during a time segment of the plurality of time segments, then normalise the deviation by an aggressiveness factor. The aggressiveness factor is comprised in the user data and corresponds to a user specific factor applied to the recommendation value. For example, the aggressiveness factor is different depending on the meal type in order to adapt to the unique user reaction depending on said meal type.

Such a characteristic allows to circumvent aggressiveness factor and therefore allows to accurately determine segment correction values and therefore accurately determine the updated correction coefficient and recommendation value.

The control device 30 also comprises a period correction unit 38 creating an updated correction coefficient for a future determined period of time based at least on the at least one correction coefficient of the determined period of time and a median of all the segment correction values. Such a characteristic allows to minimise the importance of outliers segment correction values and therefore increase the accuracy of the created updated correction coefficient and therefore accurately determine a recommendation value of the control parameter. According to a specific embodiment, the period correction unit 38 is being configured to create an updated correction coefficient based at least on the at least one correction coefficient of the determined period of time and an average of all the segment correction values. Such a characteristic allows to create an updated correction coefficient that allows to accurately determine a recommendation value of the control parameter that is typical of the period.

According to an embodiment, the period correction unit 38 is being configured to create an updated correction coefficient for a future determined period of time based at least on a segment correction value of the segment correction values and the at least one correction coefficient of the determined period of time, and wherein the updated correction coefficient is equal to the at least one correction coefficient, plus or minus 20%. In other words, the period correction unit 38 is being configured to create an updated correction coefficient for a future determined period of time based at least on a segment correction value of the segment correction values and the at least one correction coefficient of the determined period of time, and if the updated correction coefficient appears to be, for example, equal to 160% of the at least one correction coefficient, the period correction unit 38 is being configured to amend the updated correction coefficient in order for said updated correction coefficient to be equal to the at least one correction coefficient, plus or minus 20%. Such a characteristic allows to create an updated correction coefficient that is not too different from the correction coefficient and therefore does not change too much the determination of the recommendation value while still having a strong effect on said determination of the recommendation value. The purpose of such characteristics is to protect the unique user and increase his safety.

According to a specific embodiment, the period correction unit 38 is being configured to create an updated correction coefficient for a future determined period of time according to the following formula:

$$Ucc = (A * Cc) + ((1 - A) * Mcv)$$

wherein:

- Ucc is the updated correction coefficient;
- A is a weighting coefficient;
- Cc is the at least one correction coefficient; and
- Mcv is the at least one median segment correction value corresponding to the median of the segment correction values.

Such a characteristic allows to create an updated correction coefficient based on a plurality of past correction coefficients as the correction coefficient was an updated correction coefficient during the past determined period of time. Having an updated correction based on a plurality of past correction coefficients allows to determine more accurately the recommendation value.

The control device 30 comprises a recommendation unit 40 determining the recommendation value during the future determined period of time based on the updated correction coefficient, one of the plurality of amounts of a drug infused to the unique user, a plurality of physiological values of the unique user and a plurality of estimated values. An updated correction coefficient allows the recommendation unit 40 to accurately determine the recommendation value as the updated correction coefficient helps the recommendation unit 40 to adapt to the unique user changes over time. An updated correction coefficient based at least on the at least one correction coefficient of the determined period of time allows the recommendation unit 40 to accurately determine the recommendation value without implying too steep changes on the recommendation value. Too steep changes must be avoided as it could potentially reduce the time in which the unique user is in range, or in other words, the time in which the unique user physiological value such as glycemia is in an acceptable range, not in hypoglycemia or hyperglycemia. An updated correction coefficient based at least on a segment correction value of the segment correction values allows the recommendation unit 40 to accurately determine the recommendation value as each segment correction values can be created differently, time segment by time segment or depending on a specific interest of a time segment for example. The recommendation unit 40 also determines the recommendation value based on at least one of the plurality of meal sizes and one of the plurality of meal types. Such a configuration allows the recommendation unit 40 to determine an accurate recommendation value as the meal size and type has a big impact on the blood glycemia as a physiological value.

According to an embodiment, the segment correction unit 36 is being configured to create a segment correction value for each time segment wherein the segment correction value would have been able to allow the recommendation unit 40 to determine the recommendation value such as said recommendation value is sensibly equal to a value of drug, insulin, that should have been injected to the specific user during a considered time segment in order to obtain at least one physiological value of the plurality of physiological values, blood glucose, sensibly on at least one estimated of the plurality of estimated values, blood glucose target. The at least one physiological value and the at least one estimated value corresponding to the considered time segment, and wherein the at least one physiological value corresponds to blood glucose and the segment correction value is used by the recommendation unit 40 as the updated correction coefficient. Such a characteristic allows to obtain a segment correction value that would have been sensibly a perfect fit to the unique user in order to obtain physiological values on the estimated values and therefore allows the recommendation unit 40 to determine more accurately the recommendation value.

According to a specific embodiment, the segment correction value corresponds to an average of a plurality of subsegment correction values, each subsegment correction value corresponding to a sensibly perfect fit between a physiological value of the plurality of physiological values and an estimated value of the plurality of estimated values.

The segment correction unit 36 is configured to use the plurality of amounts of drug infused to the unique user in order to estimate the Insulin On Board (IOB) wherein said drug infused to the unique user is insulin. The plurality of physiological values are blood glucose values, the segment correction unit 36 is configured to use the plurality of physiological values of the unique user and at least one meal size in order to estimate a Carbohydrates On Board (COB). The COB represents the carbohydrates ingested but not yet digested, which means not visible in the blood and not raising the glycemia yet.

The segment correction unit 36 is also configured to determine a difference between the blood glycemia at the start of a time segment and the blood glycemia at the end of the same time segment and a difference between the COB at the start of the time segment and the COB at the end of the same time segment and add the ingested carbohydrates in order to determine the consumed carbohydrates. Such a configuration allows to determine the amount of insulin consumed in order to compensate for said differences.

According to a meal period embodiment wherein the correction coefficient and the updated correction coefficient are coefficients used to compensate for carbohydrates intakes of the unique user and the time segments of the interest segment list correspond to meal periods, each segment correction value is equal to the consumed insulin divided by the consumed carbohydrates. The updated correction coefficient cannot be lower than 0,02 nor higher than 0,3. Such a characteristic allows to reduce the risk of having a recommendation value too low or too high.

According to a first specific embodiment in which the plurality of physiological values of the unique user corresponds to blood glucose values of said unique user wherein the blood glucose values are measured at a regular interval, the regular interval being five minutes, the plurality of estimated values corresponds to an estimated blood glucose value of the unique user, wherein the estimated blood glucose value is estimated at the regular interval.

According to this embodiment, the segment correction unit 36 is being configured to create a segment correction value for each time segment, wherein the segment correction unit 36 is being configured to create the segment correction values based on a median of deviations between each estimated value of the plurality of estimated values and each physiological value of the plurality of physiological values during a time segment of the plurality of time segments. In this example, each deviation corresponds to the deviation between measured blood glucose value at a time t and estimated blood glucose at a time t-1. The estimated blood glucose at the time t-1 is estimated at the time t-1 and corresponds to an expected blood glucose value at the time t. Such a configuration allows the segment correction unit 36 to create a segment correction value based on the deviation between expected and actual values and therefore increases the accuracy of the recommendation value.

According to a second specific embodiment in which the plurality of physiological values of the unique user corresponds to blood glucose values of said unique user wherein the blood glucose values are measured at a regular interval, the regular interval being five minutes for example, then the plurality of estimated values corresponds to an estimated blood glucose value of the unique user, wherein the estimated blood glucose value is estimated at the regular interval. According to this embodiment, the segment correction unit 36 is being configured to create a segment correction value for each time segment, wherein the segment correction unit 36 is being configured to create the segment correction values based on a median of deviations between each estimated value of the plurality of estimated values and each physiological value of the plurality of physiological values during a time segment of the plurality of time segments. In this example, each deviation corresponds to the deviation between measured blood glucose value at a time t and a glycemia target at the time t. The glycemia target corresponds to a target blood glucose value at the time t. Such a configuration allows the segment correction unit 36 to create a segment correction value based on the deviation between expected and actual values and therefore increases the accuracy of the recommendation value.

According to a third specific embodiment, the segment correction unit 36 is being configured to create a segment correction value based on an average deviation (AVGdev), AVGdev representing an average deviation between measured blood glucose values of the plurality of physiological values during a time segment of the plurality of time segments and a blood glucose impact (BGI) of the time segment. The BGI is an estimated value of the plurality of estimated values.

The segment correction unit 36 is configured to create a segment correction value according to the following formula:

$$segment\ correction\ value\ = 1 + AVGdev/BGI$$

The segment correction unit 36 is also configured to create a segment correction value based on AVGdev and an insulin sensitivity factor (ISF) of the time segment. The ISF is an estimated value of the plurality of estimated values. Indeed, the segment correction unit 36 is configured to create a segment correction value according to the following formula:

*segment correction value = AVGdev / ISF*

According to a preferred embodiment, the segment correction unit 36 is being configured to create a segment correction value according to the following formula:

$$Scv\ = \frac{(\Sigma(AVGdev(t)\ /\ ISF(t))\ * Lrate}{Ldur}$$

wherein:

- Scv is the segment correction value;
- t represents a time point;
- Lrate represents the sampling rate of the blood glucose values during the time segment; and
- Ldur represents the time segment duration.

According to the preferred embodiment, the period correction unit 38 is being configured to create an updated correction coefficient for a future determined period of time according to the following formula:

$$updated\ correction\ coefficient\ =\ Cc*\frac{1}{3}*[Scv(i+1)\ +\ Scv(i+2)+\ Scv(i+3)]$$

wherein:

- Cc is the updated correction coefficient; and
- i represents a time segment of the plurality of time segments and i+1 represents the next considered time segment of the plurality of time segment.

The BGI is estimated based on the ISF and an insulin activity.

The recommendation unit 40 is being configured to determine the recommendation value based on the updated correction coefficient, one of the plurality of amounts of a drug infused to the unique user, one of the plurality of physiological values of the unique user and one of the plurality of estimated values and a short term coefficient. Such a configuration allows to take into account small and impermanent changes in the unique user behaviour and/or physiology. Such a configuration allows to take into account small changes during the determined period of time and therefore is useful in case of a long determined period of time.

The short term coefficient is calculated on one hand using a weighted average of *AVGdev* values and on the other hand using a maximum insulin delivery over a twenty four hours period. The weighted average of *AVGdev* values is such that when dividing the period in two equal parts, the newest values have twice the weight of the oldest values. Such a configuration allows to calculate a short term coefficient taking into account recent and impermanent changes in the unique user behaviour and/or physiology. The short term coefficient is calculated several times during the determined period of time. Such a configuration allows the control device 30 to adapt to the unique user even inside the determined period of time. The recommendation unit 40 is being configured to determine the recommendation value then multiply said recommendation value by the short term coefficient to obtain a new recommendation value. The short term coefficient cannot be greater than a high security value nor lower than a low security value. Such a configuration allows to increase the security of the control device 30. For example, the low security value is equal to 0.9 while the high security value is equal to 1.1.

According to an embodiment, the control device 30 also comprises an injection unit being configured to inject the recommendation value.

The invention also relates to a method for determining the recommendation value of a control parameter of the fluid infusion device 20, wherein the method is implemented by a control device 30 as previously described. The definitions, embodiments and technical effects previously described obviously apply, mutatis mutandis, to the method for determining the recommendation value. The method comprises the steps of:

- retrieving the user data 50;
- creating a plurality of time segments 52 in order to group user data at least based on their timestamp;
- creating a segment correction value 54 for each time segment, the segment correction values is based at least one estimated value of the plurality of estimated values and at least one physiological value of the plurality of physiological values during a time segment of the plurality of time segments, the step of creating a segment correction value 54 is performed only if the number of physiological values of the plurality of physiological values is equal or greater to a predetermined number;

creating the updated correction coefficient 56 for a future determined period of time, wherein the updated correction coefficient is based at least on a segment correction value of the segment correction values and the at least one correction coefficient of the determined period of time; and

determining the recommendation value 58 during the future determined period of time, wherein the recommendation value is determined at least based on the updated correction coefficient, one of the plurality of amounts of a drug infused to the unique user, one of the plurality of physiological values of the unique user and one of the plurality of estimated values.

Performing the step of creating a segment correction value 54 being performed only if the number of physiological values of the plurality of physiological values is equal or greater to a predetermined number allows to stop the method before the step of determining the recommendation value 58 and therefore increase the security of said method as no recommendation value is generated based on only few physiological values.

The steps are performed at a regular interval, said interval is sensibly equal to the length of the determined period of time, such a configuration allows to determine the recommendation value more accurately interval after interval while adapting to the specific user changes. When the steps are performed again, the updated correction coefficient becomes the correction coefficient while the future determined period of time becomes the determined period of time.

The step of creating the updated correction coefficient 56 implemented by the period correction unit 38 consists of creating an updated correction coefficient for a future determined period of time based at least on a segment correction value of the segment correction values and the at least one correction coefficient of the determined period of time, and wherein the updated correction coefficient is equal to the first correction coefficient of the at least one correction coefficient, plus or minus 50%. Such a characteristic allows to create an updated correction coefficient that is not too different from the first correction coefficient of the at least one correction coefficient and therefore does not change too much the determination of the recommendation value while still having a strong effect on said determination of the recommendation value.

According to an embodiment, the method also comprises an injection step consisting of injecting the recommendation value.

The invention is also related to a computer program product comprising instructions which, when the program is executed by a computer, cause the computer to carry out the steps of the method as previously described.

While exemplary embodiments of the invention have been described, it will be understood by those skilled in the art that various changes, omissions and/or additions may be made, and equivalents may be substituted for elements thereof without departing from the spirit and scope of the invention. In addition, many modifications may be made to adapt a particular situation or material to the teachings of the invention without departing from the scope thereof. Therefore, it is intended that the invention is not limited to the particular embodiments disclosed for carrying out this invention, but that the invention will include all embodiments falling within the scope of the appended claims. Moreover, unless specifically stated any use of the terms first, second, etc. do not denote any order or importance, but rather the terms first, second, etc. are used to distinguish one element from another.

**Claims**

1. A control device (30) for determining a recommendation value of a control parameter of a fluid infusion device (20), wherein the control device (30) comprises:

   - a retrieving unit (32), the retrieving unit (32) being configured to retrieve user data, each data of the user data having a timestamp and the user data being related to a unique user during a determined period of time, the user data comprising at least:

     ▪ a plurality of amounts of a drug infused to the unique user;
     ▪ a plurality of physiological values of the unique user;
     ▪ a plurality of estimated values;
     ▪ at least one correction coefficient;

       - a segmentation unit (34), the segmentation unit (34) being configured to create a plurality of time segments in order to group user data at least based on their timestamp;
       - a segment correction unit (36), the segment correction unit (36) being configured to create a segment correction value for each time segment, wherein the segment correction unit (36) is being configured to create the segment correction values based on at least one estimated value of the plurality of estimated values and at least one physiological value of the plurality of physiological values during a time segment of the plurality of time segments;
       - a period correction unit (38), the period correction unit (38) being configured to create an updated correction coefficient for a future determined period of time, wherein the period correction unit (38) is configured to create the updated correction coefficient based at least on a segment correction value of the segment correction values and the at least one correction coefficient of the determined period of time;
       - a recommendation unit (40), the recommendation unit (40) being configured to determine the recommendation value during the future determined period of time, wherein the recommendation unit (40) is being configured to determine the recommendation value at least based on the updated correction coefficient, one of the plurality of amounts of a drug infused to the unique user, one of the plurality of physiological values of the unique user and one of the plurality of estimated values.

2. A control device (30) according to claim 1, wherein the retrieving unit (32) is configured to add physiological values having a neutral value to the plurality of physiological values if the number of physiological values of the plurality of physiological values is under a predetermined number in order to obtain at least a number of physiological values equal to the predetermined number.

3. A control device (30) according to any of the claims 1 or 2, wherein the user data also comprises a plurality of meals and wherein the recommendation unit (40) is being configured to determine the recommendation value also based on at least one of the plurality of meals.

4. A control device (30) according to any of the claims 1 to 3, wherein the segmentation unit (34) is also being configured to create an interest segment list, the interest segment list comprising time segments from the plurality of time segments and being selected based on a specific interest and wherein the segment correction unit (36) is configured to create a segment correction value only for each time segment of the interest segment list.

5. A control device (30) according to any of the claims 1 to 4, wherein the period correction unit (38) is being configured to create an updated correction coefficient based at least on the at least one correction coefficient of the determined period of time and a median of all the segment correction values.

6. A control device (30) according to any of the claims 1 to 4, wherein the period correction unit (38) is being configured to create an updated correction coefficient based at least on the at least one correction coefficient of the determined period of time and an average of all the segment correction values.

7. A control device (30) according to any of the claims 1 to 6, wherein the time segments of the plurality of time segments have different lengths of time.

8. A control device (30) according to any of the claims 1 to 7, wherein the segment correction unit (36) is being configured to create a segment correction value for each time segment based on a deviation between at least one estimated value of the plurality of estimated values and at least one physiological value of the plurality of physiological values during a time segment of the plurality of time segments, then normalise the deviation by an aggressiveness factor.

9. A control device (30) according to any of the claims 1 to 8, wherein the period correction unit (38) is being configured to create an updated correction coefficient for a future determined period of time based at least on a segment correction value of the segment correction values and the at least one correction coefficient of the determined period of time, and wherein the updated correction coefficient is equal to the at least one correction coefficient, plus or minus a first predetermined percentage.

10. A control device (30) according to any of the claims 1 to 9, wherein the period correction unit (38) is being configured to create an updated correction coefficient for a future determined period of time according to the following formula:

$$Ucc = (A * Cc) + ((1 - A) * Mcv)$$

wherein:

- Ucc is the updated correction coefficient;
- A is a weighting coefficient;
- Cc is the at least one correction coefficient; and
- Mcv is the at least one median segment correction value corresponding to the median of the segment correction values.

11. Method for determining the recommendation value of a control parameter of the fluid infusion device (20), wherein the method is implemented by a control device (30) according to any of the claims 1 to 10, wherein the method comprises the steps of:

a. retrieving the user data (50);

b. creating a plurality of time segments (52) in order to group user data at least based on their timestamp;

c. creating a segment correction value (54) for each time segment, the segment correction values is based at least one estimated value of the plurality of estimated values and at least one physiological value of the plurality of physiological values during a time segment of the plurality of time segments;

d. creating the updated correction coefficient (56) for a future determined period of time, wherein the updated correction coefficient is based at least on a segment correction value of the segment correction values and the at least one correction coefficient of the determined period of time; and

e. determining the recommendation value (58) during the future determined period of time, the recommendation value is determined at least based on the updated correction coefficient, one of the plurality of amounts of a drug infused to the unique user, one of the plurality of physiological values of the unique user and one of the plurality of estimated values.

12. Method according to claim 11, wherein the step of creating a segment correction value (54) is performed only if the number of physiological values of the plurality of physiological values is equal or greater to a predetermined number.

13. The method according to any of the claims 11 or 12, wherein the steps are performed at a regular interval.

14. A computer program product comprising instructions which, when the program is executed by a computer, cause the computer to carry out the steps of the method of any of the claims 11 to 13.

[Fig. 1]

30

32

34

36

38

40

20

[Fig. 2]

50

52

54

56

58

**EP 4 354 449 A1**

Europäisches
Patentamt
European
Patent Office
Office européen
des brevets

# EUROPEAN SEARCH REPORT

Application Number

EP 23 20 2542

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2021/026004 A1 (ABBOTT DIABETES CARE INC [US]) 11 February 2021 (2021-02-11)<br>* paragraph [0063] – paragraph [0068] *<br>* paragraph [00109] – paragraph [00110] *<br>* paragraph [00164] – paragraph [00188]; figures 8A-8F *<br>* paragraph [00121] *<br>* paragraph [00134] – paragraph [00136] *<br>* paragraph [00196] *<br>* paragraph [00215] – paragraph [00253]; figure 9C *<br>* paragraph [00240] *<br>* paragraph [00418] – paragraph [00423] *<br>* paragraph [00554] – paragraph [00555] *<br>----- | 1-14 | INV.<br>G16H20/10<br>G16H20/17<br>A61B5/00 |
| X | US 2010/262434 A1 (SHAYA STEVEN A [US]) 14 October 2010 (2010-10-14)<br>* paragraph [0144] – paragraph [0147] *<br>* paragraph [0168] – paragraph [0198]; figure 5 *<br>----- | 1-14 | |
| X | US 2014/066892 A1 (KEENAN DESMOND BARRY [US] ET AL) 6 March 2014 (2014-03-06)<br>* paragraph [0273] – paragraph [0275] *<br>----- | 1-14 | TECHNICAL FIELDS SEARCHED (IPC)<br><br>A61B<br>G16H |
| X | US 2010/298685 A1 (HAYTER GARY A [US] ET AL) 25 November 2010 (2010-11-25)<br>* paragraph [0091] – paragraph [0103] *<br>* paragraph [0046] – paragraph [0053] *<br>* paragraph [0066] – paragraph [0068] *<br>----- | 1-14 | |

The present search report has been drawn up for all claims

| Place of search | Date of completion of the search | Examiner |
|---|---|---|
| Munich | 23 October 2023 | Eichenauer, Lars |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

17

## ANNEX TO THE EUROPEAN SEARCH REPORT
## ON EUROPEAN PATENT APPLICATION NO.

EP 23 20 2542

This annex lists the patent family members relating to the patent documents cited in the above-mentioned European search report.
The members are as contained in the European Patent Office EDP file on
The European Patent Office is in no way liable for these particulars which are merely given for the purpose of information.

23-10-2023

| Patent document cited in search report | Publication date | Patent family member(s) | Publication date |
|---|---|---|---|
| WO 2021026004 A1 | 11-02-2021 | AU 2020324387 A1 | 10-02-2022 |
| | | CA 3147267 A1 | 11-02-2021 |
| | | CN 114206207 A | 18-03-2022 |
| | | EP 4007523 A1 | 08-06-2022 |
| | | JP 2022543239 A | 11-10-2022 |
| | | US 2021050085 A1 | 18-02-2021 |
| | | WO 2021026004 A1 | 11-02-2021 |
| US 2010262434 A1 | 14-10-2010 | US 2010262434 A1 | 14-10-2010 |
| | | WO 2009075925 A1 | 18-06-2009 |
| US 2014066892 A1 | 06-03-2014 | AU 2013309425 A1 | 26-02-2015 |
| | | AU 2015200826 A1 | 12-03-2015 |
| | | CA 2882027 A1 | 06-03-2014 |
| | | CN 104756116 A | 01-07-2015 |
| | | CN 105999479 A | 12-10-2016 |
| | | EP 2891087 A2 | 08-07-2015 |
| | | EP 2905711 A1 | 12-08-2015 |
| | | JP 6239623 B2 | 29-11-2017 |
| | | JP 6338553 B2 | 06-06-2018 |
| | | JP 2015178044 A | 08-10-2015 |
| | | JP 2015526242 A | 10-09-2015 |
| | | KR 20150043535 A | 22-04-2015 |
| | | KR 20150050562 A | 08-05-2015 |
| | | US 2014066884 A1 | 06-03-2014 |
| | | US 2014066885 A1 | 06-03-2014 |
| | | US 2014066892 A1 | 06-03-2014 |
| | | US 2017119968 A1 | 04-05-2017 |
| | | US 2020353168 A1 | 12-11-2020 |
| | | WO 2014035570 A2 | 06-03-2014 |
| US 2010298685 A1 | 25-11-2010 | EP 2433233 A1 | 28-03-2012 |
| | | US 2010298685 A1 | 25-11-2010 |
| | | WO 2010135686 A2 | 25-11-2010 |

EPO FORM P0459

For more details about this annex : see Official Journal of the European Patent Office, No. 12/82